# FASCICULE DE BREVET EUROPEEN

(11) **EP 4 304 450 B1**
(45) Date de publication et mention de la délivrance du brevet: **19.08.2026**
(21) Numéro de dépôt: 22707703.9
(22) Date de dépôt: 23.02.2022
(51) Int. Cl.: A61B 3/11, G02C 13/00

(54) **PROCEDE DE DETERMINATION DE PARAMETRES METROLOGIQUES D'UN INDIVIDU EQUIPE D'UNE PAIRE DE LUNETTES**
VERFAHREN ZUR BESTIMMUNG VON MESSTECHNISCHEN PARAMETERN EINES MIT EINER BRILLE AUSGESTATTETEN INDIVIDUUMS
METHOD FOR DETERMINING METROLOGICAL PARAMETERS OF AN INDIVIDUAL EQUIPPED WITH A PAIR OF SPECTACLES

(30) Priorité: 10.03.2021 FR 2102349
(43) Date de publication de la demande: 17.01.2024
(73) Titulaire: LOUBSOL, 14200 Hérouville-Saint-Clair (FR)
(72) Inventeur: HANNEBICQUE, Emmanuel, 14000 Caen (FR); JOULOT, Matthieu, 75013 Paris (FR); JOULOT, Claude, 75011 Paris (FR)
(74) Mandataire: Cabinet Le Guen Maillet
(86) Numéro de dépôt international: PCT/EP2022/054522
(87) Numéro de publication internationale: WO 2022/189147

(56) Documents cités:
- EP-A1- 0 680 722
- US-A1- 2015 049 306
- US-B1- 6 535 223

## Description

### Domaine technique de l'invention

La présente invention concerne un procédé de détermination de paramètres métrologiques d'un individu équipé d'une paire de lunettes.

L'invention concerne plus particulièrement un procédé de détermination de paramètres métrologiques (écart pupillaire EP, chaque demi-écart pupillaire droit EPD, gauche EPG, chaque hauteur droite HD, gauche HG) d'un individu équipé d'une paire de lunettes pour laquelle on veut fabriquer des verres selon une prescription / ordonnance.

### Arrière-plan technique

Dès 1987, le document FR2620927 a proposé un appareil pour mesurer les différents paramètres métrologiques qui doivent être utilisés afin de positionner des verres de lunettes sur une monture, les mesures des différents paramètres étant prises chez l'opticien, directement sur le sujet portant la monture et sans immobilisation de la tête de celui-ci. Un clavier de commande permet d'effectuer des calculs sur une image capturée du sujet portant une monture qu'il a choisie et qui est placé en face d'un ensemble caméra/source lumineuse/viseur, avec la tête droite, fixant cet ensemble. L'opérateur centre l'image uniquement sur les yeux et les lunettes. La distance caméra/sujet est mesurée, ou imposée.

En 1992, le document FR2690832 a proposé un procédé d'identification du positionnement réel du centre d'une pupille d'œil par rapport à une monture de lunette donnée en vue de permettre une taille et un montage approprié des verres optiques de la monture, dans lequel on saisit en instantané l'image du visage d'un patient muni de sa monture de lunette au moyen d'un dispositif de numérisation d'images appropriées, on numérise la forme de la monture, on repère sur l'image les points extrêmes haut et bas de la monture pour tracer en surimpression sur l'image du visage les tangentes communes aux cercles droite et gauche de la monture et deux perpendiculaires aux tangentes. Selon ce procédé, on étalonne l'image notamment en calculant l'angle d'inclinaison du plan de la monture et le taux de grandissement de l'image et, on repère sur l'image ainsi étalonnée la pupille de chaque œil de manière à identifier la position réelle du centre de la pupille par rapport au cercle de monture sur une image étalonnée.

En 1993, le document FR2719463 a proposé un procédé analogue de détermination des paramètres métrologiques d'un porteur de lunettes dans lequel l'image est étalonnée par exemple par la présence dans l'image d'une mire de taille connue.

En 1994, le document EP0680722 a proposé un procédé analogue dans lequel on utilise un fichier externe représentatif de la forme du contour interne de la monture portée par le porteur de lunettes sur l'image (monture réelle ou contour réel). Un tel fichier peut être obtenu par prise d'une image vidéo sur fond discriminant ou par tout autre moyen.

Une technique dite « d'essai virtuel" d'une monture est connue pour aider les détaillants à vendre sur Internet des lunettes en fonction d'une prescription sur ordonnance afin de pallier la perte de la possibilité d'essayer des lunettes qui est un des inconvénients qui pèsent sur la vente de lunettes sur Internet.

Une fois une image faciale capturée, il est connu de combiner l'image avec des images stockées (ou un certain type de définition d'image) de montures de lunettes. L'utilisateur est alors invité à sélectionner une monture à "essayer". Une fois la paire de lunettes sélectionnée, l'image du visage stockée est superposée à une image de la monture sélectionnée. Cette image combinée est ensuite présentée à l'utilisateur afin qu'il puisse voir sa propre image portant la monture sélectionnée.

En 2000, le document US6535223 a proposé un procédé et un système pour déterminer les paramètres requis à partir d'images reçues via un réseau de communication et selon lesquels, l'utilisateur transmet une image faciale 1avec un objet de référence qui doit être d'une taille connue du serveur de mise en œuvre qui doit être facilement accessible à l'utilisateur, tel que par exemple une carte à puce de dimensions normalisées, par exemple du type dit carte de crédit ou carte de paiement. Après avoir reçu une telle image, le serveur peut superposer une image mémorisée d'une monture pour réaliser un essayage virtuel en ligne.

Toujours pour la sélection en ligne d'une monture de lunettes virtuelle parmi une pluralité de montures virtuelles, en 2009, le document FR2945365 a proposé un procédé proposant d'enregistrer une autre image du visage chaussé d'une monture étalon pour, par traitement de cette autre image, déterminer un repère d'étalonnage de la position verticale de la monture étalon sur le visage qui peut être utilisé pour la superposition de l'image de monture virtuelle sur l'image du visage, de sorte que les positions verticales relatives de la monture virtuelle et du visage d'une part, et de la monture étalon et du visage d'autre part, correspondent précisément. La monture de lunettes dite étalon peut être par exemple la propre monture du patient ou une paire de lunettes essayée en magasin qui est réputée être ajustée correctement sur son visage. L'opération d'étalonnage nécessitant l''acquisition de l'image du visage du patient chaussant la monture étalon est réalisée de préférence chez le prestataire du service de sélection en ligne de montures virtuelles, qui peut être par exemple un magasin d'optique aussi appelé opticien. L'image générée par les moyens de traitement d'images du serveur est constituée par une superposition des images respectives du visage du patient sans monture et de la monture virtuelle, la superposition d'images étant ensuite affichée sur un écran d'affichage, afin de fournir une représentation virtuelle du visage du patient chaussant la monture virtuelle.

### Résumé de l'invention

L'invention propose un procédé de détermination de paramètres métrologiques d'un individu équipé d'une paire de lunettes à partir d'au moins deux images du visage de l'individu, caractérisé en ce qu'il consiste :
- à capturer une première image du visage de l'individu sans la paire de lunettes et à capturer une deuxième image, dite image équipée, du visage de l'individu équipé de la paire de lunettes, la première image capturée ou la deuxième image contenant un objet de référence de dimensions connues ;
- à réaliser un recalage en dimensions et/ou en position et/ ou en orientation,
- soit de l'image capturée ne contenant pas l'objet de référence par rapport à l'image capturée contenant l'objet de référence,
- soit de l'image capturée contenant l'objet de référence par rapport à l'image capturée ne contenant pas l'objet de référence,
   pour obtenir une image recalée ;
- à combiner, par traitement d'images, l'image contenant l'objet de référence et ladite image recalée pour faire apparaitre les pupilles de l'individu et / ou l'objet de référence et/ou la paire de lunettes ; et
- à partir de la combinaison de l'image contenant l'objet de référence et de l'image ne contenant pas l'objet de référence, une de ces images étant recalée, à déterminer la position du centre de chaque pupille par rapport à une ligne droite horizontale tangente au contour inférieur de la paire de lunettes et par rapport à un axe vertical médian de la paire de lunettes.

Selon d'autres caractéristiques de l'invention :
- la première image capturée est une image de référence du visage de l'individu contenant un objet de référence de dimensions connues et la deuxième image capturée est une image équipée du visage de l'individu équipé de la paire de lunettes, et le procédé consiste :
   - à réaliser un recalage en dimensions et/ou en position et/ ou en orientation de la deuxième image capturée par rapport à et de la première image capturée pour obtenir une image équipée recalée ;
   - à combiner par traitement d'images l'image de référence et l'image équipée recalée pour faire apparaitre les pupilles de l'individu et / ou l'objet de référence et/ou la paire de lunettes ; et
   - à partir de la combinaison de l'image de référence et de l'image équipée recalée, déterminer la position du centre de chaque pupille par rapport à une ligne droite horizontale tangente au contour inférieur de la paire de lunettes et par rapport à un axe vertical médian de la paire de lunettes ;
le procédé consiste :
- à capturer une troisième image, dite image nue, du seul visage de l'individu ; et
- à réaliser un autre recalage en dimensions et/ou en position et/ou en orientation de l'image nue par rapport à l'image de référence pour obtenir une autre image nue recalée ;
- à partir de l'image de référence, de l'image équipée recalée et/ou de l'autre image nue recalée, pour déterminer la position du centre de chaque pupille par rapport à une ligne droite horizontale tangente au contour inférieur de la paire de lunettes et par rapport à un axe vertical médian de la paire de lunettes, le procédé consiste à :
   - déterminer au moins une dimension de l'objet de référence sur l'image de référence, et la position des pupilles sur l'image équipée ou sur l'image nue ;
   - après avoir déterminé au moins une dimension de l'objet de référence et la position des pupilles et après avoir recalé l'image équipée et/ou l'image nue par rapport à l'image de référence, on produit une image dite de mesure faisant apparaitre les pupilles de l'individu l'objet de référence et la paire de lunettes ;
   - la première image capturée est une image nue du seul visage de l'individu et la deuxième image capturée une image de référence et équipée du visage de l'individu équipé de la paire de lunettes et contenant un objet de référence de dimensions connues, et le procédé consiste :
      - à réaliser un recalage en dimensions et/ou en position et/ ou en orientation de la première image capturée par rapport à la deuxième image capturée pour obtenir une image nue recalée ;
      - à combiner, par traitement d'images, l'image de référence et équipée et l'image nue recalée pour déterminer la position du centre de chaque pupille par rapport à une ligne droite horizontale tangente au contour inférieur de la paire de lunettes et par rapport à un axe vertical médian de la paire de lunettes ; et
      - à partir de la combinaison de l'image de référence et équipée et l'image nue recalée, à déterminer la position du centre de chaque pupille par rapport à une ligne droite horizontale tangente au contour inférieur de la paire de lunettes et par rapport à un axe vertical médian de la paire de lunettes ;
      - le procédé comporte une étape consistant à déterminer au moins une dimension de l'objet de référence sur l'image de référence ou sur l'image de référence équipée et la position des pupilles sur l'image équipée ou sur l'image nue ;
      - la combinaison par traitement d'images consiste à additionner l'image non recalée contenant l'objet de référence et ladite image recalée pour faire apparaitre les pupilles de l'individu et la paire de lunettes ;
      - la combinaison par traitement d'images consiste à soustraire ladite image recalée de l'image contenant l'objet de référence pour obtenir une dite image de mesure faisant apparaitre la paire de lunettes ;
      - la paire de lunettes est pourvue de verres teintés ;
      - la paire de lunettes est une paire de lunettes d'un modèle connu préalablement identifié, ou d'un modèle connu identifié à partir d'une image capturée équipée et/ou de l'image de mesure ;
      - chaque capture d'image comporte une étape de captation d'image, au moyen d'un dispositif de prise de vues comportant un capteur d'images numériques, à une distance déterminée du visage de l'individu, et une étape de stockage de l'image captée ;
      - le dispositif de prise de vue comporte un écran d'affichage en continu de l'image à capter qui, pour chaque captation, affiche un repère de positionnement du visage de l'individu dans l'écran ;
L'invention propose aussi une méthode de commande à distance de la production d'au moins un verre de lunette destiné à être taillé pour équiper une monture d'une paire de lunettes portée par un individu déterminé, caractérisée en ce qu'elle consiste à déterminer des paramètres métrologiques de l'individu équipé de cette paire de lunettes en mettant en œuvre le procédé selon l'invention, et en ce que chaque image capturée est réalisée par ledit individu.

Le procédé selon l'invention évite à l'individu d'avoir à se rendre chez un opticien pour réaliser une prise de mesure des paramètres métrologiques mentionnés précédemment.

Avantageusement, le procédé selon l'invention peut être mis en œuvre lorsque la paire de lunettes PL est équipée de verres teintés, et ceci sans recourir à aucun moyen technique spécifique et supplémentaire tel que par exemple un éclairage spécifique émettant dans l'infrarouge.

### Brève descriptions des figures

D'autres caractéristiques et avantages de l'invention apparaitront au cours de la lecture de la description détaillée qui va suivre pour la compréhension de laquelle on se reportera aux dessins annexés dans lesquels :
[Fig.1] - la figure 1 est une représentation d'une image nue IN selon l'invention ;
[Fig.2] - la figure 2 est une représentation d'une image de référence IR selon l'invention ;
[Fig.3] - la figure 3 est une représentation d'une image équipée IE selon l'invention ;
[Fig.4] - la figure 4 est une représentation d'une image de mesure IM selon l'invention ;
[Fig.5] - la figure 5 est une représentation d'une image de référence et équipée IRE selon l'invention ;
[Fig.6] - la figure 6 est une représentation schématique permettant de définir les principaux paramètres métrologiques associés à une paire de lunettes portée par un individu.

### Description détaillée de l'invention

Pour la description de l'invention et la compréhension des revendications, on adoptera à titre non limitatif et sans référence limitative à la gravité terrestre les orientations verticale, longitudinale et transversale selon le repère V, L, T indiqué aux figures dont les axes longitudinal L et transversal T s'étendent dans un plan horizontal.

Par convention, l'axe longitudinal L est orienté de l'arrière vers l'avant.

Dans la description qui va suivre, des éléments identiques, similaires ou analogues seront désignés par les mêmes chiffres de référence.

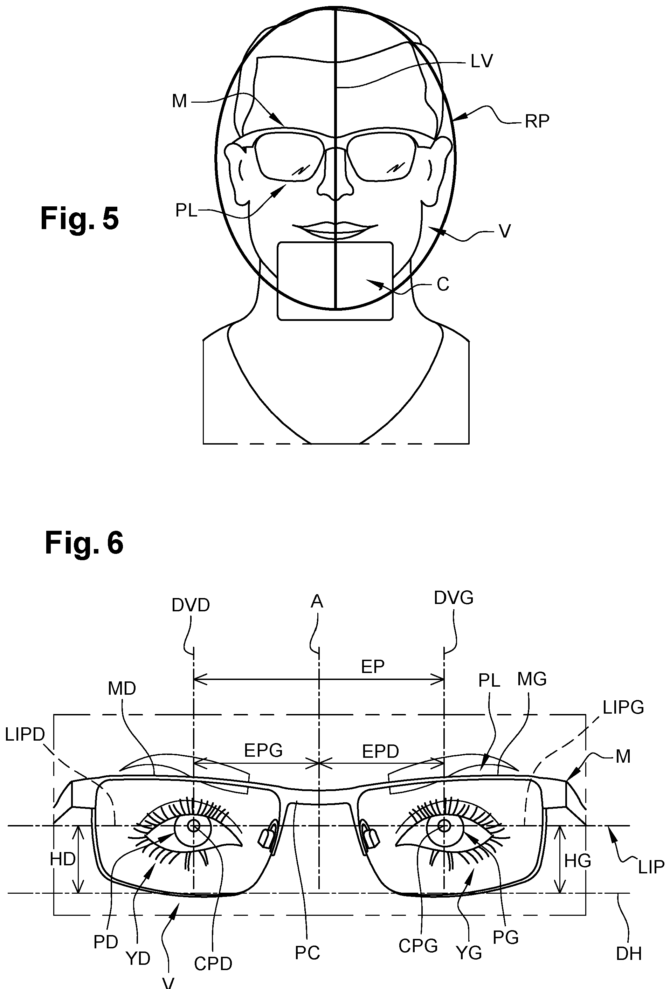

La figure 6 illustre une représentation schématique des différents paramètres métrologiques d'un individu équipé d'une paire de lunettes PL, sur laquelle on peut voir une monture de lunettes M comprenant une demi-monture gauche MG et une demi-monture droite MD, et des yeux gauche YG et droit YD du porteur de la monture M.

Pour la préparation de verres adaptés au porteur des lunettes et à une monture déterminée, il est nécessaire de connaître la position des yeux du porteur par rapport à la monture. On utilise couramment les paramètres suivants : l'écart pupillaire EP qui est la distance séparant les centres CPG et CPD des deux pupilles PG et PD, chaque demi-écart pupillaire droit EPD, gauche EPG, chaque hauteur droite HD, gauche HG).

L'écart pupillaire est égal à la somme d'un demi-écart droit EPD et d'un demi-écart gauche EPG, correspondant respectivement aux distances horizontales entre la pupille droite PG et l'axe A du nez qui est représenté en pointillés sur la figure 6, et entre l'axe A du nez et la pupille gauche PG.

On appelle pont P la distance entre les deux droites DVG et DVD verticales tangentes à la monture du côté du nez.

On appelle HG ou hauteur gauche la distance entre le centre CPG de la pupille PG de l'œil gauche YG et la droite horizontale DH tangente au bas de la monture M, et on appelle HD ou hauteur droite la distance entre le centre CPD de la pupille PD de l'œil droit YD et la droite horizontale DH.

Ainsi, à partir d'une image dite image de mesure IM comportant au moins faisant apparaitre les pupilles de l'individu et la paire de lunettes, après avoir tracé l'axe vertical A et la droite horizontale DH, il est possible de déterminer les valeurs des paramètres métrologiques EP, EPG, EPD, HG et HD, sous réserve de connaitre l'échelle de l'image de mesure IM sur laquelle on détermine les valeurs de ces paramètres.

Une technique connue pour déterminer de manière fiable l'échelle de représentation de l'image de mesure IM consiste à disposer d'une image dite image de référence IR qui est réalisée par capture d'une image du visage V du porteur de la paire de lunettes PL et qui contient un objet de référence C de dimensions connues, tel que par exemple une carte dite de paiement ou de crédit dont les dimensions sont normalisées.

Dans la suite de la description, les dénominations première image capturée, deuxième image capturée ou troisième image capturé ne définissent pas nécessairement un ordre chronologique de capture de ces différentes images.

Chaque capture d'une image comporte une étape de captation d'image au moyen d'un dispositif de prise de vues comportant un capteur d'images numériques à une distance déterminée du visage du porteur de la paire de lunettes PL, et une étape de stockage de l'image captée

Afin de pouvoir déterminer les paramètres métrologiques mentionnés précédemment, notamment à distance et sans nécessiter de mettre physiquement en présence le porteur de lunettes et un opticien, le procédé selon l'invention propose de capturer au moins une première image du visage de l'individu sans la paire de lunettes PL et de capturer au moins une deuxième, dite image équipée du visage de l'individu équipé de la paire de lunettes PL, la première image capturée ou la deuxième image contenant un objet de référence C de dimensions connues.

Ensuite, le procédé consiste à réaliser un recalage en dimensions et/ou en position et/ou en orientation d'au moins une image capturée pour obtenir au moins une image dite recalée.

Par exemple, comme cela sera expliqué plus avant, selon que l'on capture deux images IR, IE ou trois images IN, IR, IE, on recale seulement une image capturée IE par rapport à l'autre image capturée IR, ou bien une image capturée IE et une autre image capturée IN par rapport à une troisième image capturée IR.

Ce recalage est effectué par un opérateur qui, au moyen d'outils logiciels connus de traitement d'images, effectue les corrections nécessaires de ces deux images afin que les opérations ultérieures, mettant en œuvre les images précédemment capturées et recalées, soient effectuées en s'affranchissant des différences et modifications résultant des opérations préalables de captation de la première image et de la deuxième image.

Ensuite, c'est donc à partir des images capturées et des images recalées que l'on procède à une combinaison par traitement d'images pour faire apparaitre les pupilles de l'individu et / ou l'objet de référence et/ou la paire de lunettes PL.

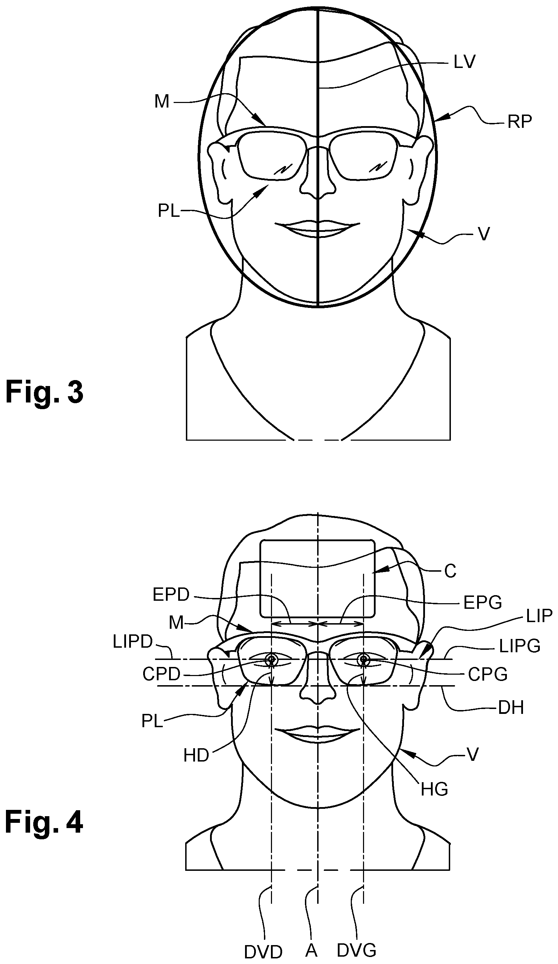

En complément, l'opérateur peut aussi produire une image de mesure IM, telle que représenté à titre d'exemple à la figure 4, faisant apparaitre les pupilles de l'individu et / ou l'objet de référence et/ou la paire de lunettes PL, qui peut être stockée.

L'opérateur disposant des images capturées et recalée(s) peut alors déterminer la position du centre CPG, CPD de chaque pupille PG, PD par rapport à une ligne droite horizontale DH tangente au contour inférieur de la paire de lunettes PL (contour de la monture ou contour des verres lorsque le bord inférieur des verres n'est pas entouré par un cercle de monture associé) et par rapport à une ligne droite verticale médiane ou axe vertical médian A de la paire de lunettes PL correspondant à l'axe du nez passant par le milieu du nez et/ou passant par le milieu du pont central PC de la monture M.

L'opérateur peut aussi tracer une ligne interpupillaire LIP passant par les deux centres CPG et CPD, et/ou deux lignes interpupillaires gauche et droite passant chacune par le centre de pupille associé CPG et CPD respectivement et étant parallèle à la ligne droite DH dite de bas de monture.

Pour déterminer la position du centre CPG, CPD de chaque pupille PG, PD par rapport à la ligne droite horizontale DH et par rapport à l'axe vertical A, le procédé consiste à déterminer la dimension de l'objet de référence C et la position des pupilles PG, PD sur l'image de référence.

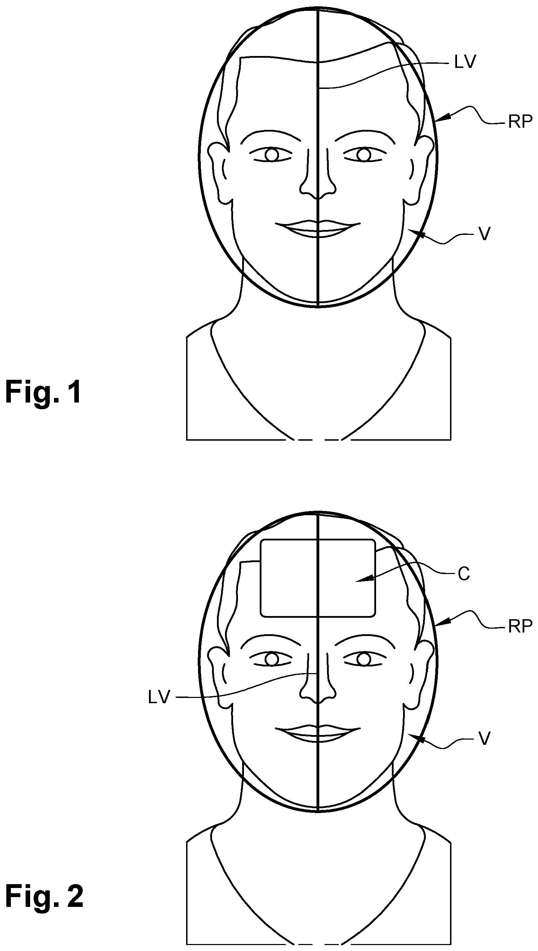

Pour faciliter la prise de vue et de garantir la plus grande homogénéité de prise de vues entre la captation des différentes images, l'individu utilise un terminal de prise de vues qui est par exemple un « smartphone » qu'il peut utiliser en mode dit «Selfie» au moyen d'une application qui fait apparaître en surimpression sur l'écran un repère RP de positionnement du visage de l'individu dans l'écran (Voir par exemple figures 1, 2 ou 3).

Les prises de vue peuvent également être effectuées avec l'aide d'une autre personne.

A titre d'exemple non limitatif le repère ou mire RP est ici un « ovale » à l'intérieur duquel, lors de chaque prise de vue ou captation d'image, l'individu adapte son visage, en modifiant notamment la distance entre sa tête et le terminal, la distance moyenne de prise de vue étant par exemple comprise entre 0,7m et 1,0m de l'objectif. Le repère RP de positionnement est de forme et de dimensions constantes sur l'écran pour la capture de toutes les images nécessaires à la mise en œuvre du procédé.

L'ovale est analogue à une ellipse symétrique par rapport à ses deux axes.

Le tracé de l'ovale peut aussi comporter une ligne droite verticale centrale LV correspondant à l'axe vertical de l'ellipse que, lors de chaque prise de vue, l'individu aligne et centre du mieux possible sur l'axe médian de son nez.

### Premier exemple de mise en œuvre du procédé

La première image capturée IR est une image du visage de l'individu contenant un l'objet de référence C de dimensions connues tel que représenté à la figure 2.

Sur cette image l'objet de référence est une carte de crédit qui est positionnée sur le front de l'individu.

La deuxième image capturée est une image dite équipée IE du visage de l'individu équipé ici de la paire de lunettes PL, tel que représenté à la figure 3.

A la figure 3, à titre non limitatif, la paire de lunettes PL est pourvue de verres teintés qui ne laissent pas apparaître les yeux de l'individu équipé de la paire de lunettes.

Selon une variante non représentée la paire de lunettes peut être pourvue de verres clairs faisant apparaître les yeux de l'individu par transparence.

On réalise alors un recalage de l'image équipée capturée IE par rapport à l'image de référence capturée IR, pour obtenir une image équipée recalée IEr (Non représentées aux figures).

Comme indiqué précédemment, après avoir déterminé la dimension de l'objet de référence C et la position des pupilles PG, PD sur l'image de référence, le procédé se poursuit en combinant par traitement d'images l'image de référence IR et l'image équipée recalée IEr pour faire apparaitre les pupilles de l'individu et / ou l'objet de référence et/ou la paire de lunettes PL.

En complément, l'opérateur peut aussi produire et stocker l'image de mesure IM faisant apparaitre les pupilles de l'individu et / ou l'objet de référence et/ou la paire de lunettes PL telle que représentée à la figure 4.

Par exemple, la combinaison consiste à additionner (superposer en transparence) l'image recalée IEr, et l'image de référence IR.

En variante, la combinaison par traitement d'images consiste à soustraire l'image de référence IR de l'image recalée IEr. Par exemple, une telle soustraction de l'image capturée de référence IR de l'image recalée IEr permet de produire une image de la monture et de l'objet de référence afin de déterminer les contours de la monture et donc sa ligne droite DH de bas de monture ainsi que les contours de l'objet de référence, et donc ses dimensions

Ensuite, à partir des images capturée de référence IR et équipée recalée IEr, on détermine la position du centre CPG, CPD de chaque pupille PG, PD par rapport à une ligne droite horizontale DH qui est tracée tangente au contour inférieur de la paire de lunettes PL et par rapport à l'axe vertical A qui est tracé en correspondance avec l'axe central du nez et/ou passant par le milieu du pont de la monture M.

Le recours à un objet de référence C de dimensions réelles connues permet de disposer ainsi avec une grande précision des valeurs des paramètres EP, EPD, EPG, HD, et HG.

L'ensemble des opérations de traitement d'images et de détermination des paramètres est de préférence réalisé de manière automatisée.

A partir de ces paramètres métrologiques de l'individu qui a réalisé les prises de vues requises au moyen de l'application dédiée qui l'a guidé lors de ces opérations, et à partir de la prescription ou ordonnance comportant les paramètres optiques des verres à réaliser pour équiper la monture M de la paire de lunettes PL portée par cet individu, il est ensuite possible de commander la production des deux verres (Ou d'au moins un verre si un seul verre est concerné par la prescription) destinés à être taillés pour équiper la monture M de la paire de lunettes PL.

Le procédé selon l'invention évite ainsi à l'individu d'avoir à se rendre chez un opticien pour réaliser une prise de mesure des paramètres métrologiques mentionnés précédemment.

Avantageusement, le procédé selon l'invention peut être mis en œuvre lorsque la paire de lunettes PL est équipée de verres teintés, et ceci sans recourir à aucun moyen technique spécifique et supplémentaire tel que par exemple un éclairage spécifique émettant dans l'infrarouge.

### Variante du premier exemple de mise en œuvre du procédé

Outre la capture d'une première image de référence IR et d'une deuxième image équipée IE, cette variante consiste à capturer une troisième image nue IN du seul visage de l'individu telle que représentée à la figure 1.

Cette troisième image capturée est une image nue IN au sens où l'individu ne porte pas la paire de lunettes et au sens où elle ne comporte pas l'objet de référence C.

De préférence, mais pas obligatoirement, l'application propose à l'individu de réaliser les trois images IN, IR puis IE, dans un ordre indifférent.

Cette variante permet de réaliser un recalage précis en dimensions et/ou en position et/ou en orientation d'une part de l'image équipée IE par rapport à la première image de référence IR et, d'autre part, de l'image nue IN par rapport à la première image de référence IR pour, comme expliqué précédemment, obtenir une image équipée recalée lEr, et une autre image nue recalée INr.

Ensuite, après avoir déterminé la dimension de l'objet de référence C et la position des pupilles PG, PD sur l'image nue IN, on réalise une combinaison par traitement d'images et on détermine les paramètres métrologiques, et éventuellement on produit l'image de mesure IM comme indiqué précédemment.

### Second exemple de mise en œuvre du procédé

Selon ce second exemple, l'individu ne capture que deux images, à savoir une première image capturée qui est une image nue IN du seul visage de l'individu et une deuxième image capturée qui est une image dite de référence et équipée IRE.

Ainsi, sur cette deuxième image capturée IRE, le visage de l'individu est équipé de la paire de lunettes PL et elle contient un objet de référence C de dimensions connues.

Un exemple d'une telle image de référence et équipée IRE est représenté à la figure 5.

A titre de variante, la carte de crédit C est ici représentée en position centrée en appui sur le menton de l'individu. Quel que soit l'exemple de mise en œuvre du procédé ou ses variantes, la position de la carte de crédit, lorsque l'objet de référence est une carte de crédit, peut être positionné indifféremment sur le front ou sur le menton de l'individu. Bien entendu, sur l'image de mesure IM, la carte de crédit sera alors positionnée au niveau du menton de l'individu.

Ensuite :
- on réalise un recalage en dimensions et/ou en position et/ ou en orientation de la première image nue capturée IN par rapport à la deuxième image de référence et équipée capturée IRE pour obtenir une image nue recalée INr ;
- on combine, par traitement d'images, l'image de référence et équipée IRE et l'image nue recalée INr pour faire apparaitre les pupilles de l'individu et / ou l'objet de référence et/ou la paire de lunettes PL ; puis
- à partir de l'image nue recalée INr et de l'image de référence et équipée IRE, on détermine la position du centre CPG, CPD de chaque pupille PG, PD par rapport à une ligne droite horizontale DH tracée sur l'image de mesure IM tangente au contour inférieur de la paire de lunettes PL et par rapport à un axe vertical médian A de la paire de lunettes PL tracé sur l'image de mesure.

En complément, l'opérateur peut aussi produire une image de mesure IM faisant apparaitre les pupilles de l'individu et / ou l'objet de référence et/ou la paire de lunettes PL, qui peut être stockée

L'objet de référence est de préférence une carte de crédit normalisée. Il est aussi possible de la remplacer par un fac-simile aux mêmes dimensions qui serait par exemple un gabarit cartonné, de préférence avec une face unie qui serait distribué par les opticiens associés à la mise en œuvre du procédé.

La production d'une image de mesure IM peut d'une part permettre de restituer visuellement à l'individu une synthèse visuelle du résultat des opérations.

Cette image de mesure IM peut aussi être fournie à l'opticien pour des opérations de vérifications ou de corrections en lui permettant notamment de déplacer la ligne droite DH, les ligne interpupillaires et/ou l'axe vertical A.

Selon une autre variante non représentée, on peut aussi réaliser un recalage en dimensions et/ou en position et/ ou en orientation de l'image IR, IRE capturée contenant l'objet de référence par rapport à l'image capturée IN, IE ne contenant pas l'objet de référence.

## Revendications

1. Procédé de détermination de paramètres métrologiques d'un individu équipé d'une paire de lunettes (PL) à partir d'au moins deux images du visage de l'individu, ce procédé consistant :
- à capturer, au moyen d'un dispositif de prise de vues, une première image (IR, IN) du visage de l'individu sans la paire de lunettes (PL) et à capturer une deuxième image, dite image équipée (IE, IRE), du visage de l'individu équipé de la paire de lunettes (PL), la première image capturée (IR) ou la deuxième image (IRE) contenant un objet de référence (C) de dimensions connues ;
- à réaliser un recalage en dimensions et/ou en position et/ ou en orientation,
- soit de l'image (IN, IE) capturée ne contenant pas l'objet de référence par rapport à l'image (IR, IRE) capturée contenant l'objet de référence,
- soit de l'image (IR, IRE) capturée contenant l'objet de référence par rapport à l'image capturée (IN, IE) ne contenant pas l'objet de référence,
pour obtenir une image recalée ;
- à combiner, au moyen d'outils logiciels de traitement d'images, l'image contenant l'objet de référence (IR, IRE) et ladite image ne contenant pas l'objet de référence, une de ces images étant recalée (IEr, INr) pour faire apparaitre les pupilles (PG, PD) de l'individu et / ou l'objet de référence (C) et/ou la paire de lunettes (PL) ; et
- à partir de la combinaison de l'image contenant l'objet de référence (IR, IRE) et de l'image ne contenant pas l'objet de référence, une de ces images étant recalée (IEr, INr), à déterminer la position du centre (CPG, CPD) de chaque pupille (PG, PD) par rapport à une ligne droite horizontale (DH) tangente au contour inférieur de la paire de lunettes (PL) et par rapport à un axe (A) vertical médian de la paire de lunettes (PL).

2. Procédé selon la revendication 1, **caractérisé en ce que** la première image capturée est une image de référence (IR) du visage de l'individu contenant un objet de référence (C) de dimensions connues et la deuxième image capturée est une image équipée (IE) du visage de l'individu équipé de la paire de lunettes (PL), et **en ce que** le procédé consiste :
- à réaliser un recalage en dimensions et/ou en position et/ ou en orientation de la deuxième image capturée (IE) par rapport à et de la première image capturée (IR) pour obtenir une image équipée recalée (IEr) ;
- à combiner par traitement d'images l'image de référence (IR) et l'image équipée recalée (IEr) pour faire apparaitre les pupilles (PG, PD) de l'individu et/ou l'objet de référence (C) et/ou la paire de lunettes (PL) ; et
- à partir de la combinaison de l'image de référence (IR) et de l'image équipée recalée (IEr), déterminer la position du centre (CPG, CPD) de chaque pupille (PG, PD) par rapport à une ligne droite horizontale (DH) tangente au contour inférieur de la paire de lunettes et par rapport à un axe vertical médian (A) de la paire de lunettes (PL).

3. Procédé selon la revendication 2, **caractérisé en ce qu'**il consiste :
- à capturer une troisième image, dite image nue (IN), du seul visage de l'individu ; et
- à réaliser un autre recalage en dimensions et/ou en position et/ou en orientation de l'image nue (IN) par rapport à l'image de référence (IR) pour obtenir une autre image nue recalée (INr).

4. Procédé selon la revendication 2 ou 3, **caractérisé en ce que**, à partir de l'image de référence (IR), de l'image équipée recalée (IEr) et/ou de l'autre image nue recalée (INr), pour déterminer la position du centre (CPG, CPD) de chaque pupille (PG, PD) par rapport à une ligne droite horizontale (DH) tangente au contour inférieur de la paire de lunettes (PL) et par rapport à un axe vertical médian (A) de la paire de lunettes (PL), le procédé consiste à :
- déterminer au moins une dimension de l'objet de référence (C) sur l'image de référence (IR), et la position des pupilles (PG, PD) sur l'image équipée (IE) ou sur l'image nue (IN).

5. Procédé selon la revendication 4 prise en combinaison avec la revendication 3, **caractérisé en ce que**, après avoir déterminé au moins une dimension de l'objet de référence (C) et la position des pupilles (PG, PD) et après avoir recalé l'image équipée (IE) et/ou l'image nue (IN) par rapport à l'image de référence (IR), on produit une image dite de mesure (IM) faisant apparaitre les pupilles (PG, PD) de l'individu l'objet de référence (C) et la paire de lunettes (PL).

6. Procédé selon la revendication 1, **caractérisé en ce que** la première image capturée est une image nue (IN) du seul visage de l'individu et la deuxième image capturée une image de référence et équipée (IRE) du visage de l'individu équipé de la paire de lunettes (PL) et contenant un objet de référence (C) de dimensions connues, et **en ce que** le procédé consiste :
- à réaliser un recalage en dimensions et/ou en position et/ ou en orientation de la première image capturée (IN) par rapport à la deuxième image capturée (IRE) pour obtenir une image nue recalée (INr) ;
- à combiner, par traitement d'images, l'image de référence et équipée (IRE) et l'image nue recalée (INr) pour déterminer la position du centre (CPG, CPD) de chaque pupille (PG, PD) par rapport à une ligne droite horizontale (DH) tangente au contour inférieur de la paire de lunettes (PL) et par rapport à un axe vertical médian (A) de la paire de lunettes (PL) ; et
- à partir de la combinaison de l'image de référence et équipée (IRE) et l'image nue recalée (INr), à déterminer la position du centre (CPG, CPD) de chaque pupille (PG, PD) par rapport à une ligne droite horizontale (DH) tangente au contour inférieur de la paire de lunettes (PL) et par rapport à un axe vertical médian (A) de la paire de lunettes (PL).

7. Procédé selon l'une des revendications 2 ou 6, **caractérisé en ce qu'**il comporte une étape consistant à déterminer au moins une dimension de l'objet de référence (C) sur l'image de référence (IR) ou sur l'image de référence équipée (IRE) et la position des pupilles (PG, PD) sur l'image équipée (IE) ou sur l'image nue (IN).

8. Procédé selon l'une quelconque des revendications précédentes, **caractérisé en ce que** la combinaison par traitement d'images consiste à additionner l'image non recalée contenant l'objet de référence (IR, IRE) et ladite image recalée (IEr, INr) pour faire apparaitre les pupilles (PG, PD) de l'individu et la paire de lunettes (PL).

9. Procédé selon la revendication 7 prise en combinaison avec la revendication 6, **caractérisé en ce que** la combinaison par traitement d'images consiste à soustraire ladite image recalée (INr) de l'image contenant l'objet de référence (IRE) pour obtenir une dite image de mesure (IM) faisant apparaitre la paire de lunettes (PL).

10. Procédé selon l'une quelconque des revendications précédentes, **caractérisé en ce que** la paire de lunettes (PL) est pourvue de verres teintés.

11. Procédé selon l'une quelconque des revendications précédentes, **caractérisé en ce que** ladite paire de lunettes (PL) est une paire de lunettes d'un modèle connu préalablement identifié, ou d'un modèle connu identifié à partir d'une image capturée équipée (IE, IRE) et/ou de l'image de mesure (IM).

12. Procédé selon l'une quelconque des revendications précédentes, **caractérisé en ce que** chaque capture d'image comporte une étape de captation d'image, au moyen d'un dispositif de prise de vues comportant un capteur d'images numériques, à une distance déterminée du visage de l'individu, et une étape de stockage de l'image captée.

13. Procédé selon la revendication précédente, **caractérisé en ce que** le dispositif de prise de vue comporte un écran d'affichage en continu de l'image à capter qui, pour chaque captation, affiche un repère (RP) de positionnement du visage de l'individu dans l'écran.

14. Méthode de commande à distance de la production d'au moins un verre de lunette destiné à être taillé pour équiper une monture (M) d'une paire de lunettes (PL) portée par un individu déterminé, **caractérisée en ce qu'**elle consiste à déterminer des paramètres métrologiques de l'individu équipé de cette paire de lunettes (PL) en mettant en œuvre le procédé selon l'une quelconque des revendications précédentes, **en ce que** chaque image capturée est réalisée par ledit individu, et **en ce que**, à partir des paramètres métrologiques de l'individu qui a réalisé les captures d'images au moyen d'une application dédiée qui l'a guidé lors de ces opérations, et à partir d'une prescription ou ordonnance comportant les paramètres optiques des verres à réaliser pour équiper la monture (M) de la paire de lunettes (PL) portée par cet individu, on commande la production d'au moins un verre destiné à être taillé pour équiper la monture (M) de la paire de lunettes (PL).

## Patentansprüche

1. Verfahren zum Bestimmen von metrologischen Parametern einer Person, die mit einer Brille (PL) ausgestattet ist, aus mindestens zwei Bildern des Gesichts der Person, dieses Verfahren bestehend aus:
- Erfassen, mittels einer Bildaufnahmevorrichtung, eines ersten Bildes (IR, IN) des Gesichts der Person ohne die Brille (PL), und Erfassen eines zweites Bildes, genannt ausgestattetes Bild (IE, IRE), des Gesichts der Person, die mit der Brille (PL) ausgestattet ist, wobei das erste aufgenommene Bild (IR) oder das zweite Bild (IRE) ein Referenzobjekt (C) mit bekannten Abmessungen enthält;
- Durchführen einer Korrektur von Abmessungen und/oder einer Position und/oder einer Ausrichtung,
- entweder von dem aufgenommenen Bild (IN, IE), das das Referenzobjekt nicht enthält, relativ zu dem aufgenommenen Bild (IR, IRE), das das Referenzobjekt enthält,
- oder von dem aufgenommenen Bild (IR, IRE), das das Referenzobjekt enthält, relativ zu dem aufgenommenen Bild (IN, IE), das das Referenzobjekt nicht enthält,
zum Erhalten eines korrigiertes Bildes;
- Kombinieren, mittels Bildverarbeitungssoftwaretools, des Bildes, das das Referenzobjekt (IR, IRE) enthält, und des Bildes, das das Referenzobjekt nicht enthält, wobei eines dieser Bilder zum Erscheinenlassen der Pupillen (PG, PD) der Person und/oder des Referenzobjekts (C) und/oder der Brille (PL) korrigiert wird (IEr, INr); und
- aus der Kombination des Bildes, das das Referenzobjekt (IR, IRE) enthält, und des Bildes, das das Referenzobjekt nicht enthält, wobei eines dieser Bilder korrigiert wird (IEr, INr), Bestimmen der Position des Zentrums (CPG, CPD) jeder Pupille (PG, PD) relativ zu einer geraden horizontalen Linie (DH), die die untere Kontur der Brille (PL) berührt, und relativ zu einer mittleren vertikalen Achse (A) der Brille (PL).

2. Verfahren nach Anspruch 1, **dadurch gekennzeichnet, dass** das erste aufgenommene Bild ein Referenzbild (IR) des Gesichts der Person ist, das ein Referenzobjekt (C) mit bekannten Abmessungen enthält, und das zweite aufgenommene Bild ein ausgestattetes Bild (IE) des Gesichts der Person ist, die mit der Brille (PL) ausgestattet ist, und **dass** das Verfahren besteht aus:
- Durchführen einer Korrektur der Abmessungen und/oder der Position und/oder der Ausrichtung des zweiten aufgenommenen Bildes (IE) relativ zu dem ersten aufgenommenen Bild (IR) zum Erhalten eines korrigierten ausgestatteten Bildes (IEr);
- Kombinieren durch eine Bildverarbeitung des Referenzbildes (IR) und des korrigierten ausgestatteten Bildes (IEr) zum Erscheinenlassen der Pupillen (PG, PD) der Person und/oder des Referenzobjekts (C) und/oder der Brille (PL); und
- aus der Kombination des Referenzbildes (IR) und des korrigierten ausgestatteten Bildes (IEr), Bestimmen der Position des Zentrums (CPG, CPD) jeder Pupille (PG, PD) relativ zu einer geraden horizontalen Linie (DH), die die untere Kontur der Brille berührt, und relativ zu einer mittleren vertikalen Achse (A) der Brille (PL).

3. Verfahren nach Anspruch 2, **dadurch gekennzeichnet, dass** es besteht aus:
- Erfassen eines dritten Bildes, genannt bloßes Bild (IN), nur von dem Gesicht der Person; und
- Durchführen einer weiteren Korrektur der Abmessungen und/oder der Position und/oder der Ausrichtung des bloßen Bildes (IN) relativ zu dem Referenzbild (IR) zum Erhalten eines weiteren korrigierten bloßen Bildes (INr).

4. Verfahren nach Anspruch 2 oder 3, **dadurch gekennzeichnet, dass** aus dem Referenzbild (IR), dem korrigierten ausgestatteten Bild (IEr) und/oder dem anderen korrigierten bloßen Bild (INr), zum Bestimmen der Position des Zentrums (CPG, CPD) jeder Pupille (PG, PD) relativ zu einer horizontalen geraden Linie (DH), die die untere Kontur der Brille (PL) berührt, und relativ zu einer mittleren vertikalen Achse (A) der Brille (PL), das Verfahren besteht aus:
- Bestimmen mindestens einer Abmessung des Referenzobjekts (C) auf dem Referenzbild (IR) und der Position der Pupillen (PG, PD) auf dem ausgestatteten Bild (IE) oder auf dem bloßen Bild (IN).

5. Verfahren nach Anspruch 4 in Kombination mit Anspruch 3,
**dadurch gekennzeichnet, dass,** nachdem mindestens eine Abmessung des Referenzobjekts (C) und die Position der Pupillen (PG, PD) bestimmt wurden und nachdem das ausgestattete Bild (IE) und/oder das bloße Bild (IN) relativ zu dem Referenzbild (IR) korrigiert wurde, ein sogenanntes Messbild (IM) erstellt wird, das die Pupillen (PG, PD) der Person, das Referenzobjekt (C) und die Brille (PL) erscheinen lässt.

6. Verfahren nach Anspruch 1, **dadurch gekennzeichnet, dass** das erste aufgenommene Bild ein bloßes Bild (IN) nur des Gesichts der Person ist und das zweite aufgenommene Bild ein Referenz- und ausgestattetes Bild (IRE) des Gesichts der Person ist, die mit der Brille (PL) ausgestattet ist, und ein Referenzobjekt (C) mit bekannten Abmessungen enthält, und **dass** das Verfahren besteht aus:
- Durchführen einer Korrektur der Abmessungen und/oder der Position und/oder der Ausrichtung des ersten aufgenommenen Bildes (IN) relativ zu dem zweiten aufgenommenen Bild (IRE) zum Erhalten eines korrigierten bloßen Bildes (INr);
- Kombinieren, durch die Bildverarbeitung, des Referenz- und ausgestatteten Bildes (IRE) und des korrigierten bloßen Bildes (INr) zum Bestimmen der Position des Zentrums (CPG, CPD) jeder Pupille (PG, PD) relativ zu einer geraden horizontalen Linie (DH), die die untere Kontur der Brille (PL) berührt, und relativ zu einer mittleren vertikalen Achse (A) der Brille (PL); und
- aus der Kombination des Referenz- und ausgestatteten Bildes (IRE) und des korrigierten bloßen Bildes (INr), Bestimmen der Position des Zentrums (CPG, CPD) jeder Pupille (PG, PD) relativ zu einer horizontalen geraden Linie (DH), die die untere Kontur der Brille (PL) berührt, und relativ zu einer mittleren vertikalen Achse (A) der Brille (PL).

7. Verfahren nach einem der Ansprüche 2 oder 6,
**dadurch gekennzeichnet, dass** es einen Schritt aufweist, der aus dem Bestimmen mindestens einer Abmessung des Referenzobjekts (C) auf dem Referenzbild (IR) oder auf dem ausgestatteten Referenzbild (IRE) und der Position der Pupillen (PG, PD) auf dem ausgestatteten Bild (IE) oder auf dem bloßen Bild (IN) besteht.

8. Verfahren nach einem der vorstehenden Ansprüche,
**dadurch gekennzeichnet, dass** die Kombination durch die Bildverarbeitung aus einem Zusammenführen des nicht korrigierten Bildes, das das Referenzobjekt (IR, IRE) enthält, und des korrigierten Bildes (IEr, INr) zum Erscheinenlassen der Pupillen (PG, PD) der Person und die Brille (PL) besteht.

9. Verfahren nach Anspruch 7 in Kombination mit Anspruch 6,
**dadurch gekennzeichnet, dass** die Kombination durch die Bildverarbeitung aus einem Abziehen des korrigierten Bildes (INr) von dem Bild, das das Referenzobjekt (IRE) enthält, zum Erhalten eines Messbildes (IM) besteht, das die Brille (PL) erscheinen lässt.

10. Verfahren nach einem der vorstehenden Ansprüche,
**dadurch gekennzeichnet, dass** die Brille (PL) mit getönten Gläsern versehen ist.

11. Verfahren nach einem der vorstehenden Ansprüche,
**dadurch gekennzeichnet, dass** die Brille (PL) eine Brille eines zuvor identifizierten bekannten Modells oder eines bekannten Modells ist, das aus einem ausgestatteten aufgenommenen Bild (IE, IRE) und/oder dem Messbild (IM) identifiziert wird.

12. Verfahren nach einem der vorstehenden Ansprüche,
**dadurch gekennzeichnet, dass** jede Bilderfassung einen Schritt zum Erfassen eines Bildes mittels einer Bildaufnahmevorrichtung, die einen digitalen Bildsensor aufweist, in einem bestimmten Abstand von dem Gesicht der Person, und einen Schritt zum Speichern des aufgenommenen Bildes aufweist.

13. Verfahren nach dem vorstehenden Anspruch,
**dadurch gekennzeichnet, dass** die Bildaufnahmevorrichtung einen Bildschirm für eine kontinuierliche Anzeige des aufzunehmenden Bildes aufweist, der für jede Erfassung eine Markierung (RP) zum Positionieren des Gesichts der Person auf dem Bildschirm anzeigt.

14. Verfahren für eine Fernsteuerung der Erstellung mindestens eines Brillenglases, das dazu bestimmt ist, zum Ausstatten einer Fassung (M) einer Brille (PL) zugeschnitten zu werden, die durch eine bestimmte Person getragen wird, **dadurch gekennzeichnet, dass** es aus dem Bestimmen von metrologischen Parametern der Person, die mit dieser Brille (PL) ausgestattet ist, durch Implementieren des Verfahrens nach einem der vorstehenden Ansprüche besteht, **dass** jedes aufgenommene Bild durch die Person aufgenommen wird, und **dass** aus den metrologischen Parametern der Person, die die Bildaufnahmen mittels einer speziellen Anwendung durchgeführt hat, die sie während dieser Vorgänge angeleitet hat, und aus einer Verschreibung oder einem Rezept, das die optischen Parameter der Gläser aufweist, die zum Ausstatten der Fassung (M) der Brille (PL) hergestellt werden sollen, die durch diese Person getragen wird, die Erstellung mindestens eines Glases gesteuert wird, das dazu bestimmt ist, zum Ausstatten der Fassung (M) der Brille (PL) zugeschnitten zu werden.

## Claims

1. A method for determining metrological parameters of an individual equipped with a pair of glasses (PL) from at least two images of the face of the individual, this method consisting of:
- capturing, by means of an image capture device, a first image (IR, IN) of the face of the individual without the pair of glasses (PL) and capturing a second image, a so-called equipped image (IE, IRE), of the face of the individual equipped with the pair of glasses (PL), the first captured image (IR) or the second image (IRE) containing a reference object (C) of known dimensions;
- perfoming an adjustment in dimensions and/or position and/or orientation of,
- either the captured image (IN, IE) not containing the reference object with respect to the captured image (IR, IRE) containing the reference object,
- or the captured image (IR, IRE) containing the reference object with respect to the captured image (IN, IE) not containing the reference object,
in order to obtain an adjusted image;
- combining, by means of image processing software tools, the image containing the reference object (IR, IRE) and said image not containing the reference object, one of these images being adjusted (IEr, INr) to show the pupils (PG, PD) of the individual and/or the reference object (C) and/or the pair of glasses (PL); and
- from the combination of the image containing the reference object (IR, IRE) and the image not containing the reference object, one of these images being adjusted (IEr, INr), determining the position of the center (CPG, CPD) of each pupil (PG, PD) with respect to a horizontal straight line (DH) tangential to the lower contour of the pair of glasses (PL) and with respect to a median vertical axis (A) of the pair of glasses (PL).

2. The method according to claim 1, **characterized in that** the first image captured is a reference image (IR) of the face of the individual containing a reference object (C) of known dimensions and the second image captured is an equipped image (IE) of the face of the individual equipped with the pair of glasses (PL), and **in that** the method consists of:
- performing an adjustement in dimensions and/or position and/or orientation of the second captured image (IE) with respect to the first captured image (IR) in order to obtain an adjusted equipped image (IEr);
- combining, by image processing, the reference image (IR) and the adjusted equipped image (IEr) to show the pupils (PG, PD) of the individual and/or the reference object (C) and/or the pair of glasses (PL); and
- from the combination of the reference image (IR) and the adjusted equipped image (IEr), determining the position of the center (CPG, CPD) of each pupil (PG, PD) with respect to a horizontal straight line (DH) tangential to the lower contour of the pair of glasses and with respect to a median vertical axis (A) of the pair of glasses (PL).

3. The method according to claim 2, **characterized in that** it consists of:
- capturing a third image, a so-called bare image (IN), of the individual's face only; and
- performing another adjustement in dimensions and/or position and/or orientation of the bare image (IN) with respect to the reference image (IR) in order to obtain another adjusted bare image (INr).

4. The method according to claim 2 or 3, **characterized in that,** from the reference image (IR), the adjusted equipped image (IEr) and/or the other adjusted bare image (INr), in order determine the position of the center (CPG, CPD) of each pupil (PG, PD) with respect to a horizontal straight line (DH) tangential to the lower contour of the pair of spectacles (PL) and with respect to a median vertical axis (A) of the pair of glasses (PL), the method consists of:
- determining at least one dimension of the reference object (C) on the reference image (IR), and the position of the pupils (PG, PD) on the equipped image (IE) or on the bare image (IN).

5. The method according to claim 4 taken in combination with claim 3, **characterized in that,** after having determined at least one dimension of the reference object (C) and the position of the pupils (PG, PD) and after having adjusted the equipped image (IE) and/or the bare image (IN) with respect to the reference image (IR), a so-called measurement image (IM) is produced, showing the pupils (PG, PD) of the individual, the reference object (C) and the pair of glasses (PL).

6. The method according to claim 1, **characterized in that** the first image captured is a bare image (IN) of the individual's face only and the second image captured is a reference and equipped image (IRE) of the individual's face equipped with the pair of glasses (PL) and containing a reference object (C) of known dimensions, and **in that** the method consists of:
- adjusting in dimensions and/or position and/or orientation the first captured image (IN) with respect to the second captured image (IRE) in order to obtain a recalibrated bare image (INr);
- combining, by image processing, the reference and equipped image (IRE) and the adjusted bare image (INr) to determine the position of the center (CPG, CPD) of each pupil (PG, PD) with respect to a horizontal straight line (DH) tangential to the lower contour of the pair of glasses (PL) and with respect to a median vertical axis (A) of the pair of glasses (PL); and
- from the combination of the reference and equipped image (IRE) and the adjusted bare image (INr), determining the position of the center (CPG, CPD) of each pupil (PG, PD) with respect to a horizontal straight line (DH) tangential to the lower contour of the pair of glasses (PL) and with respect to a median vertical axis (A) of the pair of glasses (PL).

7. The method according to one of claims 2 or 6, **characterized in that** it comprises a step consisting of determining at least one dimension of the reference object (C) on the reference image (IR) or on the equipped reference image (IRE) and the position of the pupils (PG, PD) on the equipped image (IE) or on the bare image (IN).

8. The method according to any one of the preceding claims,
**characterized in that** the image processing combination consists of adding the non-adjusted image containing the reference object (IR, IRE) and said adjusted image (IEr, INr) to show the pupils (PG, PD) of the individual and the pair of glasses (PL).

9. The method according to claim 7 taken in combination with claim 6, **characterized in that** the combination by image processing consists of subtracting said adjusted image (INr) from the image containing the reference object (IRE) in order to obtain a so-called measurement image (IM) showing the pair of glasses (PL).

10. The method according to any one of the preceding claims,
**characterized in that** the pair of glasses (PL) is provided with tinted lenses.

11. The method according to any one of the preceding claims,
**characterized in that** said pair of glasses (PL) is a pair of glasses of a previously identified known model, or of a known model identified from an equipped captured image (IE, IRE) and/or from the measurement image (IM).

12. The method according to any one of the preceding claims,
**characterized in that** each image capture comprises a step of capturing an image, by means of an image capture device comprising a digital image sensor, at a determined distance from the individual's face, and a step of storing the captured image.

13. The method according to the preceding claim, **characterized in that** the image capture device comprises a screen for continuous display of the image to be captured which, for each capture, displays a mark (RP) for positioning the individual's face in the screen.

14. The method for remotely controlling the production of at least one spectacle lens intended to be shaped to equip a frame (M) of a pair of spectacles (PL) worn by a given individual, **characterized in that** it consists of determining metrological parameters of the individual equipped with this pair of spectacles (PL) by implementing the method according to any one of the preceding claims, **in that** each image captured is taken by said individual, and **in that,** on the basis of the metrological parameters of the individual who has captured the images by means of a dedicated application which has guided the individual during these operations, and on the basis of a prescription comprising the optical parameters of the lenses to be produced to equip the frame (M) of the pair of spectacles (PL) worn by this individual, the production of at least one lens intended to be shaped to equip the frame (M) of the pair of spectacles (PL) is requested.
